# EUROPÄISCHE PATENTANMELDUNG

(11) **EP 1 281 393 A2**
(43) Veröffentlichungstag der Anmeldung: **05.02.2003**
(21) Anmeldenummer: 02016687.2
(22) Anmeldetag: 26.07.2002
(51) Int. Cl.: A61K 7/48, A01N 25/04, A01N 25/34

(54) **Hygieneartikel welcher mit einem alkoholischen Gel befeuchtet ist**

(30) Priorität: 04.08.2001 DE 10138457
(71) Anmelder: Bode Chemie GmbH & Co., 22525 Hamburg (DE)
(72) Erfinder: Möllers, Ulrich, Dr., 22395 Hamburg (DE); Rudolf, Marco, Dr., 22299 Hamburg (DE); Holling, Jaana, 22523 Hamburg (DE); Knieler, Roland, Dr., 21227 Benderstorf (DE)

(57) **Zusammenfassung**

Hygieneartikel zum Erfrischen, Desodorieren oder Desinfizieren, umfassend ein geformtes, saugfähiges Material, welches mit einem alkoholischen Gel befeuchtet ist.

## Beschreibung

Die vorliegende Erfindung betrifft kosmetische und dermatologische Tücher, welche mit alkoholischen Gelen befeuchtet sind. Insbesondere betrifft die Erfindung mit alkoholischen Gelen getränkte eingesiegelte Tücher zum Erfrischen, Desodorieren oder mikrobiziden Dekontaminieren, vorzugsweise mit alkoholischen Gelen getränkte textile Tücher.

Reinigungstücher werden im Haushalt, aber auch im gewerblichen Bereich in großer Zahl eingesetzt. Sie werden beispielsweise zur Reinigung des Körpers angewendet, z. B. als Toilettenpapier, Taschentücher und Servietten, in der Babypflege, zum Abschminken oder zum Reinigen oder Trocknen der Hände, aber auch generell zum Säubern von Oberflächen und Geräten, z. B. in der Küche oder im Laborbetrieb. Meist werden die Reinigungstücher als trockene Papiere verwendet, in jüngster Zeit finden aber auch immer häufiger feuchte Papiere Verwendung. Die Befeuchtung soll dabei in der Regel dazu dienen, eine bessere Reinigung und darüber hinaus auch eine Pflege der zu reinigenden Flächen zu erzielen.

Getränkte Tücher sind handliche Vehikel für kosmetische oder dermatologische Präparate und erlauben unter anderem eine effiziente und hautschonende Reinigung und Pflege besonders auch in der Abwesenheit von (fließendem) Wasser.
Dabei besteht der eigentliche Gebrauchsgegenstand aus zwei Komponenten:
a) einem glatten oder auch oberflächenstrukturierten trockenen Tuch, welches sowohl aus wasserlöslichen als auch aus wasserunlöslichen Materialien wie Papier und/oder unterschiedlichsten Mischungen aus Natur- oder Kunstfasern aufgebaut sein kann und
b) einer niederviskosen Tränkungslösung, beispielsweise auf der Basis von Ölen und/oder Wasser sowie Emulsionen, alkoholische Lösungen und dergleichen mehr.

Um ein Austrocknen derartiger feuchter Tücher zu verhindern, werden diese üblicherweise in einer feuchtigkeitsdichten bzw. in einer für die Tränkungslösung undurchlässigen Umhüllung verpackt (eingesiegelt). Der Stand der Technik kennt einzeln oder stapelweise verpackte Tücher. Einzeln verpackte Tücher sind beispielsweise die sogenannten "Erfrischungstücher", welche in Flugzeugen oder Restaurants zum Reinigen der Hände ausgegeben werden. Solche Tücher sind vor allem auf Reisen praktisch, weil sie eine für die Anwendung geeignete Menge Präparat portioniert enthalten und man dementsprechend kein unnötiges Gewicht im Reisegepäck mit sich führen muß.

Alkohole - wie Ethanol, Propan-1-ol und Propan-2-ol - werden in der Kosmetik und Dermatologie in großem Umfang eingesetzt, beispielsweise als Haar- oder Gesichtswasser, in Form von Eau de Cologne, Franzbranntwein, als Antiseptikum und dergleichen. Die Funktion der Alkohole ist dabei sehr vielfältig und unterschiedlich: Sie können z. B. als Lösungsmittel für dermatologisch wirksame Substanzen und/oder Hautlipide dienen sowie als die Durchblutung fördernde Wirksubstanz oder als mikrobizides Agens. Ferner wird durch hohe Mengen an Alkoholen auch ein Kühleffekt erzielt, da diese beim Verteilen der Formulierung auf der Haut spontan verdunsten. Alkoholische Lösungen sind dementsprechend sehr vorteilhaft als Tränkungslösungen für Tuchmaterialien zu verwenden.

Allerdings weisen herkömmliche niederviskose Imprägnier- bzw. Tränkungslösungen für Tuchmaterialien einige Nachteile auf. Bei der Herstellung getränkter Tücher mit Hilfe von niedrigviskosen Tränklösungen ist es z. B. besonders wesentlich und damit produktionsaufwendig, die Menge der Tränklösung ausreichend zu dosieren. Ist die Dosierung zu niedrig, wird der erwünschte - reinigende, desinfizierende usw. - Effekt nicht erzielt, ist die Dosierung zu hoch, tropft das Präparat beim Öffnen der Verpackung wegen der niedrigen Viskosität der Tränklösungen aus dem Träger heraus. Durch die heraustropfende Tränklösung wird dabei nicht selten - und besonders unangenehm auf Reisen - die Kleidung verunreinigt, ferner wird das Präparat auf diese Weise sinnlos vergeudet.

Es war Aufgabe der vorliegenden Erfindung, alkoholische Imprägnier- bzw. Tränkungslösungen zum Aufbringen auf Trägermaterialien zu finden, die die Nachteile des Standes der Technik nicht zeigen.

Es war überraschend und für den Fachmann nicht vorauszusehen, daß ein
Hygieneartikel zum Erfrischen, Desodorieren oder Desinfizieren, umfassend ein geformtes, saugfähiges Material, welches mit einem alkoholischen Gel befeuchtet ist,
den Nachteilen des Standes der Technik abhelfen würde.

Die erfindungsgemäßen Gele enthalten vorteilhaft 20 bis 99 Gew.-% der Alkohole Ethanol, Propan-1-ol, Propan-2-ol entweder einzeln oder in beliebigen Mischungen voneinander. Die Viskosität der erfindungsgemäßen Gele wird vorzugsweise aus dem Bereich von 3.000 bis 20.000 mPa·s gewählt.

Die Vorteile der vorliegenden Erfindung zeigen sich vor allem bei Trägermaterialien mit mittlerem bis schlechtem Flüssigkeitsaufnahmevermögen: Während bei Materialien mit einem sehr guten Flüssigkeitsaufnahmevermögen - wie z. B. einem Schwamm - Unterschiede in der aufgenommenen Menge an Tränkungsmedium kaum noch nachweisbar sind, kann in Trägermaterialien mit mittlerem bis schlechtem Flüssigkeitsaufnahmevermögen die aufgenommene Menge an Tränkungsmedium durch die Verwendung der erfindungsgemäßen Gele erheblich gesteigert werden, ohne daß die Tücher dabei die Nachteile des Standes der Technik zeigen würden.

Darüber hinaus ist das Aufnahmevermögen abhängig von der Viskosität des Gels. Das Trägermaterial kann umso mehr Gel aufnehmen, je höher dessen Viskosität ist. Während bei der Verwendung von wässrigen Systemen als Tränkungslösungen vor allem Trägermaterialien mit hydrophilen Gruppen ein hohes Wasseraufnaufnahmevermögen aufweisen, entfällt dieser Effekt für alkoholischen Zubereitungen weitgehend, da diese überwiegend über die Struktur der Kavitäten aufgenommen werden.

Es war daher umso überraschender, daß bereits leichte Viskositätserhöhungen der alkoholischen Zubereitungen zu einer deutlich erhöhten Aufnahme im Trägermaterial führen.

Zur Bildung erfindungsgemäßer alkoholischer Gele werden Verdickersysteme benötigt, die aus einem positiv oder negativ geladenem Polymer und einem entgegengesetzt geladenen "Neutralisationsmittel" bestehen. Derartige Verdickersysteme sind an sich bekannt. Die Auswahl des geeigneten Verdickers bzw. des am besten geeigneten Verdickersystems hängt von der Art des/der verwendeten Alkohols bzw. Alkohole und dessen/deren Konzentration ab. Am gebräuchlichsten sind Systeme von verzweigter Polyacrylsäure (z. B. Carbopol EDT 2020 oder Carbopol Ultrez 10 der Fa. Goodrich) und einem basischen Neutralisationsmittel, vorzugsweise einem organischen Amin. Solche Amine sind beispielsweise Diisopropylamin, Triethanolamin, Tetrahydroxypropylethylendiamin oder ethoxiliertes Cocosamin (mit 15 Ethoxyeinheiten).

Je höher die angestrebte Viskosität und je höher die Alkoholkonzentration, desto größer ist die Menge des benötigten Verdickersystems.

Die Firma Goodrich gibt zum Beispiel folgende Empfehlung für die Herstellung alkoholischer Gele, welche vorteilhaft im Sinne der vorliegenden Erfindung zu verwenden sind:

| **Alkoholgehalt** | **Gelbildner** | **Neutralisationsmittel** |
|---|---|---|
| bis 20 Vol.-% Alkohol | Polyacrylsäure (Carbopol) | Natriumhydroxyd |
| bis 30 Vol.-% Alkohol | Polyacrylsäure (Carbopol) | Kaliumhydroxyd |
| bis 60 Vol.-% Alkohol | Polyacrylsäure (Carbopol) | Triethanolamin |
| bis 60 Vol.-% Alkohol | Polyacrylsäure (Carbopol) | Trisamino* |
| bis 80 Vol.-% Alkohol | Polyacrylsäure (Carbopol) | Aminopropylmethan |
| bis 90 Vol.-% Alkohol | Polyacrylsäure (Carbopol) | Neutrol TE** |
| bis 90 Vol.-% Alkohol | Polyacrylsäure (Carbopol) | Diisopropanolamin |
| bis 90 Vol.-% Alkohol | Polyacrylsäure (Carbopol) | Triisopropanolamin |
| bis 90 Vol.-% Alkohol | Polyacrylsäure (Carbopol) | Ethomeen C-25 *** |

| | | |
|---|---|---|
| * 2-Amino-2-(Hydroxymethyl)-1,3-Propandiol | | |
| ** Tetrahydroxypropylethylendiamin | | |
| *** Ethoxyliertes Cocosamin (15 EO) | | |

Da die polymeren Gelbildner nicht in die Haut einziehen, sondern einen Film bilden, ist es vorteilhaft im Sinne der vorliegenden Erfindung ein Verdickersystem zu verwenden, von dem möglichst wenig benötigt wird. Die bevorzugte Menge an Verdickersystem (bestehend aus Polymer plus Neutralisationsmittel) beträgt 0,3 bis 5,0 Gew.-%, bezogen auf das Gesamtgewicht des alkoholischen Gels.

Wenn bei der vorgesehenen Anwendung die Aspekte Reinigung, Kühlung oder "Durchblutungsförderung" im Vordergrund stehen, ist es erfindungsgemäß bevorzugt, Ethanol in einer Konzentration von 30 bis 60 Gew.-% einzusetzen, bezogen auf das Gesamtgewicht des alkoholischen Gels. Steht eine antiseptische Anwendung im Vordergrund, so sind höhere Alkoholkonzentrationen von 60 bis 99 Gew.-%, bezogen auf das Gesamtgewicht des alkoholischen Gels, erforderlich. Es ist insbesondere vorteilhaft im Sinne der vorliegenden Erfindung bei dieser Anwendung auch Propanole einzusetzen.

Die alkoholischen Gele können weitere kosmetisch wirksame Bestandteile enthalten wie beispielsweise Antitranspirantien, insbesondere Aluminiumchlorid, Aluminiumchlorhydrat, Zirkoniumchlorid oder Zirkoniumchlorid(Aluminiumchlorid-Mischsalze.

Femer können die erfindungsgemäßen Gele pflegende Öle und Fette enthalten, wie z. B. Ester aus gesättigten und/oder ungesättigten verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen mit einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat Isononylisononaniat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner können solche pflegenden Öle vorteilhaft gewählt werden aus der Gruppe der verzweigten und/oder unverzweigten Kohlenwasserstoffe, der Silikonöle, der Dialkylether, der Gruppe der gesättigten oder ungesättigten, verzweigten oder unverzweigten Alkohole sowie der Fettsäuretriglyceride, namentlich der Glycerintriester gesättigter und/oder ungesättigter, verzweigter und/oder unverzweigter Alkancarbonsäuren einer Kettenlänge von 8 bis 24 insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Olivenöl, Sonnenblumenöl, Sojaöl, Erdnussöl, Rapsöl, Mandelöl, Palmöl, Kokosöl, Palmkernöl und ähnlichen Ölen.

Besonders vorteilhafte alkoholische Gele im Sinne der vorliegenden Erfindung erhalten ferner Antioxidantien als Zusatz- oder Wirkstoffe. Erfindungsgemäß enthalten die Gele vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Als Antioxydantien kommen in Frage: z. B. die Gruppe der Aminosäuren (z. B. Glycin, Histidin, Tyrosin, Tryptophan) und deren Derivate, Imidazole (z. B. Urocaninsäure) und deren Dervate, Peptide wie D,L-Camosin, L-Carnosin und deren Dervate (z. B. Anserin), Carotinoide, Carotine (z. B. α-Carotin, β-Carotin, Lycopin) und deren Derivate, Liponsäure und deren Derivate (z. B. Dihydroliponsäure, Aurothioglucose, Propylthiouracil und andere Thiole (z. B. Thioredoxin, Glutathion, Cystein, Cystin, Cystamin, und deren Glycoyl-, N-Acetyl-, Methyl-, Ethyl-, Propyl-, Amyl-, Butyl-, Lauryl-, Palmitoyl-, Oleyl-, γ-Linoleyl-, Cholesteryl-, und Glycerylester) sowie deren Salze, Dilaurylthiodipropionat, Distearylthiodipropionat, Thiodipropionsäure, und deren Derivate (Ester, Ether, Peptide, Lipide, Nucleotide, Nucleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen, verträglichen Dosierungen (z. B. pmol bis µmol/kg) ferner (Metall-) chelatoren (z. B. α-Hydroxyfettsäuren, Palmitinsäure, Phytinsäure, Lactoferrin) α-Hydroxysäuren, (z. B. Citronensäure, Milchsäure, Apfelsäure) Huminsäure, Gallensäure, Gallenextrakte, Bilirubin, Biliverdin, EDTA, EGTA, und deren Derivate ungesättigte Fettsäuren und deren Derivate (z. B. γ-Linolensäure, Linolsäure, Ölsäure), Folsäure und Derivate, Alanindiessigsäure, Flavonoide, Polyphenole, Catechine, Ubichinon und Ubichinol und deren Derivate, Vitamin C und Derivate (z. B. Ascorbylpalmitat, Mg-Ascorbylphosphat, Ascorbylacetat), Tocopherole und Derivate (z.B. Vitamin-E-Acetat), sowie Coniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguarajetsäure, Trihydroxybutyrophenon, Harnsäure und deren Derivate, Mannose und deren Derivate, Zink und dessen Derivate (z. B. ZnO und ZnSO₄), Selen und dessen Derivate (z. B. Selenmethionin) Stilbene und deren Derivate (z. B. Stilbenoxid, trans-Stilbenoxid und die erfindungsgemäß geeigneten Derivate (Salze, Ester, Ether, Zucker, Nukleotide, Nukleoside Peptide und Lipide) dieser Wirkstoffe.

Die Menge der Antioxidantien (eine oder mehrere Verbindungen) in den Zubereitungen beträgt vorzugsweise 0,001 bis 3,0 Gew. % bezogen auf das Gesamtgewicht des Gels. Aus den genannten Verbindungen werden diejenigen bevorzugt, die in der entsprechenden Rezeptur löslich sind.

Als Träger können gewebte, gewirkte oder offenporig geschäumte Materialien verwendet werden. Erfindungsgemäß vorteilhaft sind Träger aus Cellulose oder Cellulosederivaten (wie zum Beispiel Papier) oder solche auf der Basis von textilen Geweben (wie Baumwolle, Wolle, Leinen, Jute oder Nessel) oder solche auf der Basis von synthetischen Fasern (wie Polyester, Polyamid, Polyvinylacetat, Polyacrylnitril) oder auf der Basis von offenporigen Schaumstoffen (wie Polyurethanschaum, Naturkautschukschaum oder Synthesekautschukschaum).

Bevorzugte Trägermaterialien sind trockene Tücher, welche beispielsweise wie Papier und/oder aus unterschiedlichsten Mischungen aus Natur- oder Kunstfasern oder deren Gemischen aufgebaut sind. Derartige Tücher können gegebenenfalls vorteilhaft (makroskopisch) geprägt sein.

Vorteilhafte Tücher bestehen z. B. aus wasserstrahlverfestigten und/oder wasserstrahlgeprägten Vliesen (Spunlaced-Material). Sofern die Vliese Prägungen aufweisen, können diese jedes gewünschte Muster haben. Die zu treffende Auswahl richtet sich zum einen nach nach der Eigenart des aufzubringenden Gels, zum andern nach der vorgesehenen Anwendung.

Große Kavitäten an der Tuchoberfläche und im Tuch erlauben die Aufnahme größerer Mengen an Gel und ergeben zusätzlich einen Massage Effekt, wenn mit solchen Tüchern die Haut behandelt wird.

Ferner sind auch gewebte Tücher und Papiere vorteilhafte Trägermaterialien im Sinne der vorliegenden Erfindung.

Vorteilhafte Tücher im Sinne der vorliegenden Erfindung haben eine Reißkraft von:

| | | [N/50mm] |
|---|---|---|
| im trockenen Zustand | Längsrichtung | >60, vorzugsweise >80 |
| | Querrichtung | >20, vorzugsweise >30 |
| im getränkten Zustand | Längsrichtung | >4, vorzugsweise >60 |
| | Querrichtung | >10, vorzugsweise >20 |
| | | |

| Die Dehnfähigkeit des Tuches beträgt vorzugsweise | | |
|---|---|---|
| im trockenen Zustand | Längsrichtung | 15 % bis 100 %, bevorzugt |
| | | 20 % und 50 % |
| | Querrichtung | 40 % bis 120 %, bevorzugt |
| | | 50 % und 85 % |
| im getränkten Zustand | Längsrichtung | 15 % bis 100 %, bevorzugt |
| | | 20 % und 40 % |
| | Querrichtung | 40 % bis 120 %, bevorzugt |
| | | 50 % und 85 % |

Es hat sich als vorteilhaft herausgestellt für das Tuch, wenn dieses ein Gewicht von 35 bis 120 g/m², vorzugsweise von 40 bis 60 g/m², hat (gemessen bei 20 °C ± 2 °C und bei einer Feuchtigkeit der Raumluft von 65 % ± 5 % für 24 Stunden).

Sofern es sich bei dem Trägermaterial um ein Tuch handelt, beträgt dessen Dicke vorzugsweise 0,4 bis 1,5 mm, insbesondere 0,6 bis 0,9 mm.

Als Ausgangsmaterialien für gewebte oder nicht gewebte Tücher können generell alle organischen oder anorganischen Faserstoffe auf natürlicher oder synthetischer Basis verwendet werden. Beispielhaft seien Viskose, Baumwolle, Jute, Hanf, Sisal, Seide, Wolle, Polypropylen, Polyester (z. B. Polyethylentherephthalat, PET), Aramid, Nylon, Polyvinylderivate wie Polyvinylacetat, Polyurethan, Polylactid, Polyhydroxyalkanoat, Celluloseester, und Polyethylen sowie gegebenenfalls auch mineralische Fasern wie Glasfasern oder Kohlenstofffasem angeführt, die alle entweder allein oder im Gemisch verwendet werden können. Die vorliegende Erfindung ist nicht auf die genannten Materialien beschränkt, sondern es können eine Vielzahl weiterer Fasern zur Tuchbildung eingesetzt werden.

In einer besonders vorteilhaften Ausführungsform bestehen die Fasern möglichst zu 100 % aus Viskose. Besonders vorteilhaft sind auch Fasern aus hochfesten Polymeren wie Polyamid, Polyester und/oder gerecktem Polyethylen.

Darüber hinaus können die Fasern auch eingefärbt sein, um die optische Attraktivität des Tuches betonen und/oder erhöhen zu können. Die Fasern können zusätzlich UV-Stabilisatoren und/oder Konservierungsmittel enthalten.

Die zur Bildung des Tuches eingesetzten Fasern weisen vorzugsweise eine Wasseraufnahmerate von mehr als 60 mm/[10 min], (gemessen mit dem EDANA Test 10.1-72), insbesondere mehr als 80 mm/[10 min] auf.

Femer weisen die zur Bildung des Tuches eingesetzten Fasern vorzugsweise ein Wasseraufnahmevermögen von mehr als 5g/g, gemessen mit dem EDANA-Test 10.1-72, insbesondere mehr als 8 g/g auf.

Die nachfolgenden Beispiele sollen die erfindungsgemäßen Tränkungslösungen verdeutlichen, ohne sie einzuschränken. Die Zahlenwerte in den Beispielen bedeuten Gewichtsprozente, bezogen auf das Gesamtgewicht der jeweiligen Zubereitungen. An diesen Beispielen soll ferner gezeigt werden, dass an einem Träger mit mittlerem Wasseraufnahmevermögen die Aufnahme von alkoholischen Zubereitungen durch Gelbildung/Viskositätserhöhung dramatisch steigern lässt:

### Beispiele:

Ein rechteckiges Viscosevlies mit einer Ausdehnung 18,5 x 17,5 cm², einem Flächengewicht von 75 g/m² nach DIN EN 29073-1, einer Dicke 0,769 mm nach DIN EN 29073-1, einer Höchstzugkraft, längs 98 N/5cm nach DIN EN 29073-3, einer Höchstzugkraft quer von 69 N/5cm nach DIN EN 29073-3, einer Dehnung längs von 17% nach DIN EN 29073-3 und einer Dehnung quer von 43% nach DIN EN 29073-3 wurde
a) mit Ethanol, 96%ig
b) mit einem alkoholischen Gel von einer Viskosität von 2946 mPa·s, Rezeptur 1
c) mit einem alkoholischen Gel von einer Viskosität von 7300 mPa·s, Rezeptur 2
versehen und bestimmt, wieviel Alkohol bzw. Gel das Vlies aufnimmt, bevor etwas heraustropft
a) Ethanol: 8 ml, tropft nicht, 9 ml tropft beim Anwenden, 10 ml tropft im gefalteten Zustand ohne Anwendung
b) ethanolisches Gel, Viskosität 2946 mPa.s: 8 ml, Vlies nicht ausreichend getränkt, 10 ml, Vlies ist gut getränkt, tropft nicht, 14 ml: zu viel Gel auf dem Tuch, Tropft nicht, 30 ml, zu viel Gel, fängt an zu tropfen.
c) ethanolisches Gel, Viskosität 7300 mPa.s, tropft nicht bei der Aufnahme von 32 ml was deutlich zu viel ist, weil zu feucht.

| **Rezeptur 1 (nach INCI)** | |
|---|---|
| Alcohol denat. | 85,86 Gew. Teile |
| Aqua | 10,88 Gew. Teile |
| Acrylates /C10-30 Alkylacrylate Crosspolymer | 0.30 Gew. Teile |
| Glycerol | 0,59 Gew. Teile |
| PEG-40-Hydrogenated Castor Oil | 0,50 Gew. Teile |
| Propylene Glycol | 0,60 Gew. Teile |
| Panthenol | 0,67 Gew. Teile |
| Tetrahydroxy propylethylene diamine | 0,60 Gew. Teile |

| **Rezeptur 2 (nach INCI)** | |
|---|---|
| Alcohol denat | 65,657 Gew. Teile |
| Aqua | 31,993 Gew. Teile |
| Carbomer | 0,250 Gew. Teile |
| Glycerol | 1,00 Gew. Teile |
| Isopropylalcohol | 0,50 Gew. Teile |
| PEG 7 Glycerylcocoate | 0,30 Gew. Teile |
| Parfüm | 0,10 Gew. Teile |
| Tetrahydroxypropylethylendiamine | 0,20 Gew. Teile |

## Patentansprüche

1. Hygieneartikel zum Erfrischen, Desodorieren oder Desinfizieren, umfassend ein geformtes, saugfähiges Material, welches mit einem alkoholischen Gel befeuchtet ist.

2. Hygieneartikel nach Anspruch 1, **dadurch gekennzeichnet, dass** das geformte, saugfähige Material ein Tuch, einen Schwamm, einen Bausch, einen Flausch darstellt.

3. Hygieneartikel nach einem der Ansprüche 1 oder 2, **dadurch gekennzeichnet, dass** das geformte, saugfähige Material aus Cellulose, Cellulosederivaten, Baumwolle, Papier, Vlies und/oder Polyurethanschwamm besteht.

4. Hygieneartikel nach einem der Ansprüche 1 bis 3, **dadurch gekennzeichnet, dass** das alkoholische Gel Ethanol, Propan-1-ol und/oder Propan-2-ol entweder einzeln oder im Zweiergemisch oder im Dreiergemisch enthält.

5. Hygieneartikel nach einem der Ansprüche 1 bis 4, dadurch gekenzeichnet, dass der Alkoholgehalt des alkoholischen Gels 20 bis 99 Gew.-% beträgt, bezogen auf das Gesamtgewicht des Gels.

6. Hygieneartikel nach einem der Ansprüche 1 bis 5, **dadurch gekennzeichnet, dass** das alkoholische Gel eine Viskosität von 3000 bis 20 000 mPa·s aufweist.

7. Hygieneartikel nach einem der Ansprüche 1 bis 6, **dadurch gekennzeichnet, dass** die alkoholischen Gele ferner kosmetische und/oder antitranspirante und/oder mikrobizide Inhaltsstoffe enthalten.

8. Hygieneartikel nach einem der Ansprüche 1 bis 7, **dadurch gekennzeichnet, dass** der Artikel in für das Gel und deren Inhaltsstoffe undurchlässiges Material eingeschlossen ist.
